(19) Europäisches Patentamt
European Patent Office
Office européen des brevets

(11) **EP 3 351 286 A1**

(12) **EUROPEAN PATENT APPLICATION**
published in accordance with Art. 153(4) EPC

(43) Date of publication:
25.07.2018 Bulletin 2018/30

(51) Int Cl.:
*A61M 37/00* (2006.01)   *A61M 5/142* (2006.01)
*A61M 5/168* (2006.01)

(21) Application number: 16846097.0

(22) Date of filing: 20.07.2016

(86) International application number:
PCT/JP2016/071271

(87) International publication number:
WO 2017/047224 (23.03.2017 Gazette 2017/12)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 14.09.2015 JP 2015181054

(71) Applicant: Terumo Kabushiki Kaisha
Tokyo 151-0072 (JP)

(72) Inventors:
• YAEGASHI Mitsutoshi
Aomori 030-0861 (JP)
• SUGAWARA Yoshihisa
Ashigarakami-gun
Kanagawa 259-0151 (JP)

(74) Representative: TBK
Bavariaring 4-6
80336 München (DE)

(54) **DEVICE FOR ADMINISTERING DRUG SOLUTION**

(57)   An embodiment of the present invention includes: a pump part that delivers and administers a drug solution held in a reservoir to a live body; a flow rate sensing part that senses the flow rate of the solution delivered by the pump part; and a drive unit that controls the drive state of the pump part based on the sensed flow rate. The flow rate sensing part includes: a heat-generating element disposed in a conduit line in which the drug solution is delivered; a first temperature sensing element disposed in the conduit line upstream of the heat-generating element; a second temperature sensing element disposed in the conduit line downstream of the heat-generating element; and an arithmetic processing unit that calculates the flow rate within the conduit line based on the difference between the temperatures sensed by the first and second temperature sensing elements.

## FIG. 2

EP 3 351 286 A1

## Description

Technical Field

The present invention relates to a device for administering a drug-solution, for example, a portable drug-solution administration device for administering insulin into a body.

Background Art

A portable insulin administration device has been developed which automatically administers insulin, which is a drug solution for controlling blood glucose, subcutaneously to a diabetic patient. A small-sized pump is built in the portable insulin administration device, and a drug solution (insulin) stored in a reservoir is administered to the patient by driving the pump.

When administering a drug solution such as insulin, it is necessary to administer the drug solution at a low flow rate with high accuracy. For accurate administration at a low flow rate, a conventional insulin administration device adopting a syringe pump system in which a stepping motor and a decelerator are combined has been the mainstream.

Patent Literature 1 describes an example of a drug transport device applicable to an insulin administration device, and an example of using a piezoelectric pump using a piezoelectric element as a pump is described.

Citation List

Patent Literature

Patent Literature 1: JP 2002-126092 A

Summary of Invention

Technical Problem

A conventional syringe pump system with a stepping motor and a decelerator combined has a disadvantage of difficulty of downsizing and of a large operating noise and thus the system is not suitable for a drug-solution administration device which is small in size as a portable type. For this reason, application of a miniaturized pump such as a piezoelectric pump as described in Patent Literature 1 has been studied. However, a miniaturized pump such as a piezoelectric pump has a problem that the accuracy thereof at a low flow rate is insufficient.

An object of the present invention is to provide a drug-solution administration device suitable for miniaturization, which enables administration of a drug solution with high accuracy at a low flow rate.

Solution to Problem

A drug-solution administration device of the present invention includes a pump part for feeding a drug solution held in a reservoir and administering the drug solution to a live body, a flow rate sensing part for detecting a flow rate of the solution fed by the pump part, and a drive unit for controlling a drive state of the pump part on the basis of the flow rate detected by a flow rate sensing part.

The flow rate sensing part includes a heat-generating element disposed in a conduit line for sending a drug solution, a first temperature sensing element disposed in a conduit line on the upstream side of the heat-generating element, a second temperature sensing element disposed in a conduit line on the downstream side of the heat-generating element, and an arithmetic processing unit for calculating the flow rate in the conduit line based on the difference between temperatures detected by the first and second temperature sensing elements.

According to the drug-solution administration device of the present invention, even when the flow rate of the drug solution produced by driving of the pump part is low, the flow rate sensing part can detect the accurate mount of drug solution administered, and the drive unit can control the drive state of the pump part with high accuracy based on the detected accurate amount of drug solution administered. Therefore, according to the drug-solution administration device of the present invention, a drug solution can be administered at a low flow rate with high accuracy.

Brief Description of Drawings

Fig. 1 is a perspective view illustrating an internal configuration example of a drug-solution administration device according to an embodiment of the present invention.
Fig. 2 is a cross-sectional view illustrating a configuration example of a flow rate sensing part provided in the drug-solution administration device according to an embodiment of the present invention.
Fig. 3 is a circuit diagram illustrating a circuit configuration example connected to a temperature sensing element of the flow rate sensing part according to an embodiment of the present invention.
Fig. 4 is a circuit diagram illustrating a configuration example of a drive circuit of a heat-generating element of the flow rate sensing part according to an embodiment of the present invention.
Fig. 5 is a characteristic diagram illustrating an example of a flow rate of a drug solution by a pump part according to an embodiment of the present invention.
Fig. 6 is a characteristic diagram illustrating an example of a flow rate sensing state according to an embodiment of the present invention.

Fig. 7 is a characteristic diagram illustrating an example of the relationship between detected voltage and the flow rate according to an embodiment of the present invention.

Fig. 8 is a characteristic diagram illustrating an example of the relationship between the detected voltage and the temperature according to an embodiment of the present invention.

Fig. 9 is a characteristic diagram illustrating in detail an example of the relationship between the detected voltage and the temperature according to an embodiment of the present invention.

Fig. 10 is a timing chart illustrating a driving example of the pump part according to an embodiment of the present invention.

Fig. 11 is a characteristic diagram illustrating a control example at a specific temperature according to an embodiment of the present invention.

Fig. 12 is a cross-sectional view illustrating a configuration example of the flow rate sensing part in a modification example according to an embodiment of the present invention.

Fig. 13 is a cross-sectional view taken along line A-A in Fig. 12.

Description of Embodiments

Hereinafter, a drug-solution administration device according to an embodiment of the present invention will be described with reference to the accompanying drawings.

[1. Overall Configuration of Drug-solution Administration Device]

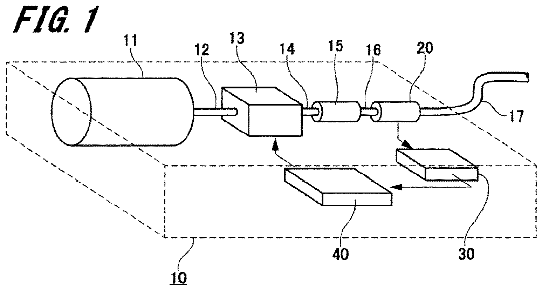

Fig. 1 shows an internal configuration of a drug-solution administration device 10. The drug-solution administration device 10 is housed in a compact casing as a portable type and used as an insulin administration device.

As shown in Fig. 1, the drug-solution administration device 10 includes a reservoir 11 for storing a drug solution (here, insulin) . A pump part 13 is connected to the reservoir 11 via a conduit line 12.

As the pump part 13, a piezoelectric pump is used. The piezoelectric pump vibrates a diaphragm disposed inside by using a piezoelectric element and delivers the drug solution held in the reservoir 11 to a conduit line 14. A valve which keeps the liquid feeding direction of the drug solution constant is built in the pump part 13.

When the pump part 13 does not contain a valve, an on-off valve may be disposed in the conduit line 12 between the reservoir 11 and the pump part 13 to be opened and closed in conjunction with the vibration of the diaphragm in the pump part 13.

A microneedle valve 15 is connected to the conduit line 14 to which the drug solution is sent from the pump part 13. The liquid feeding state for the drug solution from the pump part 13 is pulsative, and the microneedle valve 15 acts to suppress this pulsation. A flow rate sensing part 20 is connected to a conduit line 16 to which a drug solution whose pulsation is suppressed by the microneedle valve 15 is delivered. It should be noted that the microneedle valve 15 may not be disposed between the conduit lines 14 and 16.

A conduit line 17 drawn out to the outside of the drug-solution administration device 10 is connected to the flow rate sensing part 20, and the drug solution is injected into a live body via the conduit line 17. The configuration for injecting the drug solution into the body will be omitted.

Respective elements (a first temperature sensing element 22, heat-generating element 23, second temperature sensing element 24 to be described later) incorporated in the flow rate sensing part 20 are connected to an arithmetic processing unit 30. The arithmetic processing unit 30 detects the flow rate of the drug solution passing through the flow rate sensing part 20 based on the state of each element. Information on the flow rate detected by the arithmetic processing unit 30 is supplied to a drive unit 40.

The drive unit 40 determines the necessary amount of the drug solution administered according to the operation mode of the drug-solution administration device 10, and controls the drive state of the pump part 13. At this time, the drive state of the pump part 13 is corrected and the amount of the drug solution administered is controlled to an accurate state based on the information on the flow rate detected by the arithmetic processing unit 30. When the drive unit 40 controls the pump part 13 formed of a piezoelectric pump, the liquid feeding amount is controlled by setting the driving voltage and the driving frequency.

[2. Configuration of Flow Rate Sensing Part]

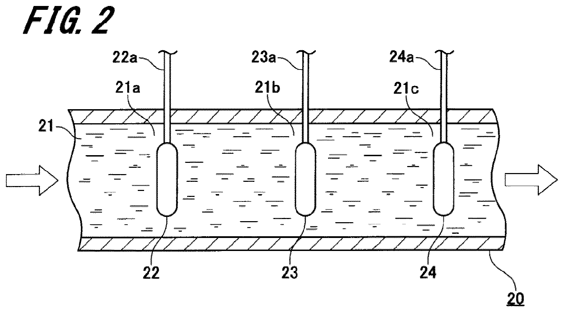

Fig. 2 shows the configuration of the flow rate sensing part 20.

The flow rate sensing part 20 includes a conduit line 21 in which the first temperature sensing element 22, the heat-generating element 23, and the second temperature sensing element 24 are arranged.

The first temperature sensing element 22, the heat-generating element 23, and the second temperature sensing element 24 are arranged from the upstream side of the conduit line 21 to be substantially equally spaced. That is, the first temperature sensing element 22 is disposed at the most upstream side position 21a inside the conduit line 21. Then, the heat-generating element 23 is disposed at a position 21b that is separated by a predetermined distance from the position 21a where the first temperature sensing element 22 is disposed. Further, the second temperature sensing element 24 is disposed at a position 21c that is separated by a predetermined distance from the position 21b where the heat-generating element 23 is disposed. As the first temperature sensing element 22 and the second temperature sensing element 24, thermistors whose resistance values vary with temperature are used, for example. Also for the heat-generating element 23, a thermistor for a heater is used. In the following description, the first temperature sensing element 22 and the second temperature sensing element 24 are referred to as an upstream-side temperature sensing element and a downstream-side temperature sensing element.

Then, the arithmetic processing unit 30 connected to the flow rate sensing part 20 drives the heat-generating element 23 to generate heat and detects the flow rate inside the conduit line 21 in accordance with the temperatures detected by the upstream-side temperature sensing element 22 and the downstream-side temperature sensing element 24.

The details of the principle of detection of the flow rate from the temperatures detected by the upstream-side temperature sensing element 22 and the downstream-side temperature sensing element 24 of the flow rate sensing part 20 will be described later, but this can be explained briefly as follows. For example, when the heat-generating element 23 heats the drug solution in the conduit line 21 in a state where there is no flow rate in the conduit line 21, the temperatures detected by two temperature sensing elements 22 and 24 having the same distance from the heat-generating element 23 are equal. On the other hand, when some flow rate is generated in the conduit line 21, the temperatures detected by the two temperature sensing elements 22 and 24 are different from each other. Utilizing this principle, the flow rate sensing part 20 detects the flow rate from the difference between temperatures detected by the two temperature sensing elements 22 and 24.

[3. Circuit Connected to Each Element]

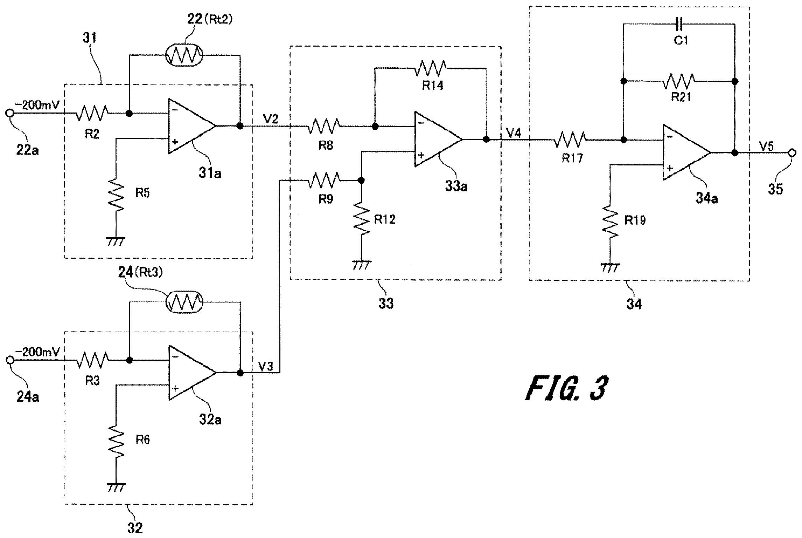

Fig. 3 is a circuit example of the arithmetic processing unit 30 connected to the upstream-side temperature sensing element 22 and the downstream-side temperature sensing element 24. The resistance value Rt2 of the upstream-side temperature sensing element 22 is converted into a voltage value by a first voltage conversion circuit 31 and the resistance value Rt3 of the downstream-side temperature sensing element 24 is converted into a voltage value by a second voltage conversion circuit 32.

With regard to the configuration of the first voltage conversion circuit 31, the upstream-side temperature sensing element 22 formed of a thermistor is connected between the inverting input terminal (-) and the output terminal of an operational amplifier 31a constituting the first voltage conversion circuit 31. A predetermined voltage (for example, -200 mV) is applied from a terminal 22a to the inverting input terminal (-) of the operational amplifier 31a via a resistor R2.

The non-inverting input terminal (+) of the operational amplifier 31a is connected to the ground via a resistor R5. The voltage V2 obtained at the output terminal of the operational amplifier 31a has a voltage value proportional to the resistance value Rt2 of the upstream-side temperature sensing element 22.

The configuration of the second voltage conversion circuit 32 is the same as that of the first voltage conversion circuit 31. That is, the downstream-side temperature sensing element 24 formed of a thermistor is connected between the inverting input terminal (-) and the output terminal of an operational amplifier 32a constituting the second voltage conversion circuit 32. A predetermined voltage (for example, -200 mV) is applied to the inverting input terminal (-) of the operational amplifier 32a from a terminal 24a via a resistor R3.

The non-inverting input terminal (+) of the operational amplifier 32a is connected to the ground through a resistor R6. The voltage V3 obtained at the output terminal of the operational amplifier 32a has a voltage value proportional to the resistance value Rt3 of the downstream-side temperature sensing element 24.

The voltage V2 obtained by the first voltage conversion circuit 31 and the voltage V3 obtained by the second voltage conversion circuit 32 are input to a differential amplifier circuit 33 so that a differential voltage V4 between both the voltages is acquired. That is, the voltage V2 obtained by the first voltage conversion circuit 31 is applied to the inverting input terminal (-) of an operational amplifier 33a constituting the differential amplifier circuit 33 via a resistor R8. The voltage V3 obtained by the second voltage conversion circuit 32 is applied to the non-inverting input terminal (+) of the operational amplifier 33a via a resistor R9.

The connection point between the resistor R9 and the non-inverting input terminal (+) of the operational amplifier 33a is connected to the ground via a resistor R12, and the inverting input terminal (-) and the output terminal of the operational amplifier 33a are connected by a resistor R14. The amplification factor of the output voltage V4 of the differential amplifier circuit 33 is determined according to the resistance value of each resistor. A detailed example of the amplification factor will be described later.

The differential voltage V4 obtained by the differential amplifier circuit 33 is applied to a low-pass filter 34, and the voltage V5 from which the high frequency component which is noise has been removed is output to an output terminal 35. Specifically, the low-pass filter 34 is composed of an operational amplifier 34a, resistors R17, R19, and R21, and a capacitor C1 connected to the operational amplifier 34a. In the low-pass filter 34, the voltage V5 from which high-frequency noise has been removed can be obtained by the action of the resistor R21 and the capacitor C1.

The arithmetic processing unit 30 calculates the average of the voltage V5 to obtain the flow rate.

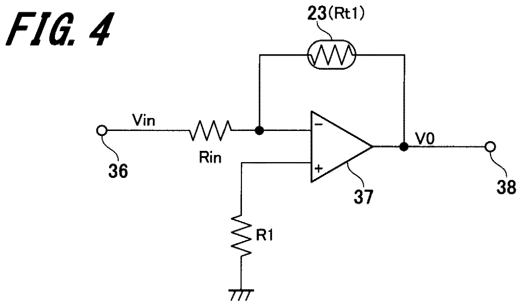

Fig. 4 is a diagram showing an example of a circuit connected to the heat-generating element 23. The circuit shown in Fig. 4 is also provided in the arithmetic processing unit 30. The voltage Vin applied to a terminal 36 is impressed to the inverting input terminal (-) of an operational amplifier 37 via a resistor Rin. The non-inverting input terminal (+) of the operational amplifier 37 is connected to the ground through a resistor R1.

Then, the heat-generating element 23 is connected between the inverting input terminal (-) and the output terminal of the operational amplifier 37. The voltage V0 output to the output terminal of the operational amplifier 37 is obtained at a terminal 38. The voltage V0 obtained at the terminal 38 is used for detecting the liquid temperature at the position of the heat-generating element 23 during a heat dissipation period (idle period of heat generation) to be described later. In the description of the operation to be described later, the resistance value of the heat-generating element 23 is referred to as Rt1.

[4. Example of Feeding State by Pump Part]

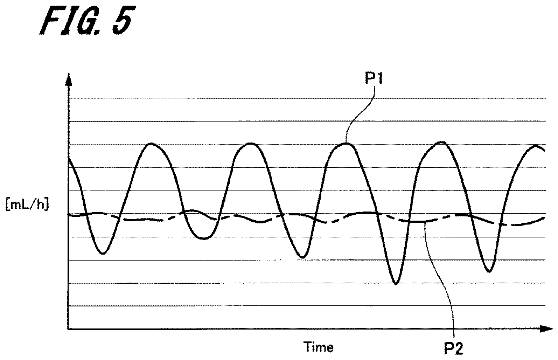

Fig. 5 shows an example of a liquid feeding state by the pump part 13. In Fig. 5, the vertical axis represents the flow rate and the horizontal axis represents time. A characteristic P1 shown in Fig. 5 indicates the fluctuation in the flow rate of the pump part 13 alone. In the pump part 13 using a piezoelectric pump, the characteristic P1 of the flow rate of a single unit fluctuates by about $\pm 15\%$ with respect to the flow rate desired to be set originally due to the influence of temperature or the like.

A characteristic P2 shown in Fig. 5 indicates the fluctuation of the flow rate at the output portion of the microneedle valve 15. By providing the microneedle valve 15, the fluctuation amount of the flow rate can be reduced as illustrated.

[5. Principle of Detection of Flow Rate by Flow Rate Sensing Part]

Next, the principle of detection of the flow rate using each element arranged in the flow rate sensing part 20 will be described.

For example, when the heat-generating element 23 heats the drug solution in the conduit line 21 in a state in which there is no flow rate inside the conduit line 21 of the flow rate sensing part 20, the temperatures detected by the two temperature sensing elements 22 and 24 having the same distance from the heat-generating element 23 are equal to each other. On the other hand, when there is some flow rate in the conduit line 21, the temperatures detected by the two temperature sensing elements 22 and 24 are different from each other.

Here, suppose that the resistance value of the upstream-side temperature sensing element 22 made of a thermistor whose resistance value varies with temperature is Rt2, the resistance value of the downstream-side temperature sensing element 24 is Rt3, and the constant current I is supplied to each of the temperature sensing elements 22 and 24, the output voltages V2 and V3 of the first voltage conversion circuit 31 and the second voltage conversion circuit 32 are expressed by the following equations (1) and (2). The current I is selected in the range of 0.02 mA to 0.05 mA, for example. In this case, the current I is 0.02 mA.

$$V2 = I \cdot Rt2 \cdots (1)$$

$$V3 = I \cdot Rt3 \cdots (2)$$

Further, if the amplification factor of the output voltage V4 of the differential amplifier circuit 33 to which the voltages V2 and V3 are input is G, the voltage V4 is expressed by the following equation (3). The equation (3) can also be expressed as equation (4). The amplification factor G is 10 times, for example.

$$V4 = G (V3 - V2) \cdots (3)$$

$$V4 = G \cdot I (Rt3 - Rt2) \cdots (4)$$

The amplification factor G is set by the ratio of the resistance values of the resistors R8 and R14. Namely, G=R14/R8. However, it is assumed that the resistors R8 and R9 have the same resistance value and the resistors R12 and R14 have the same resistance value.

With respect to the temperature T [°C] within a specific temperature range, the resistance value Rt of the thermistor is expressed by the following equation (5).

$$Rt = -a \cdot T + b \cdots (5)$$

Here, a and b are positive constants. In this example, a negative temperature coefficient (NTC) thermistor having a characteristic in which the resistance value decreases with increase in temperature is used as the thermistor.

Therefore, the voltage V4 can be expressed as the following equation (6) by substituting the equation (5) into the equation (4). In the equation (6), T2 is the temperature of the upstream-side temperature sensing element 22, and T3 is the temperature of the downstream-side temperature sensing element 24.

$$V4 = G \cdot I \cdot a (T3 - T2) \cdots (6)$$

From this equation (6), it can be seen that the output voltage V4 of the differential amplifier circuit 33 is proportional to the temperature difference between the upstream-side temperature sensing element 22 and the downstream-side temperature sensing element 24. The voltage V5 obtained by removing noise from the voltage V4 with the low-pass filter 34 is the output of the arithmetic processing unit 30 connected to the flow rate sensing part 20.

Meanwhile, the heat-generating element 23 is driven so as to repeat heat generation and heat dissipation at a constant frequency. For example, the element repeats, about two or three times, a process taking 20 seconds per one cycle including 16 seconds of heat generation and 4 seconds of heat dissipation. By changing the current that flows through the heat-generating element 23 for the heat generation period and the heat dissipation period, both sufficient heat generation and accurate temperature measurement with ignorable influence of self-heating can be performed. That is, the temperature measurement of the drug solution using the heat-generating element 23 is per formed during the heat dissipation period.

By repeating the heat generation and heat dissipation in this way, excessive heating of the drug solution is prevented. To be specific, the arithmetic processing unit 30 performs control to generate heat so as to keep the temperature rise due to heat generation within 2 [°C] and so as to stop the heat generation when the temperature rise likely to exceed.

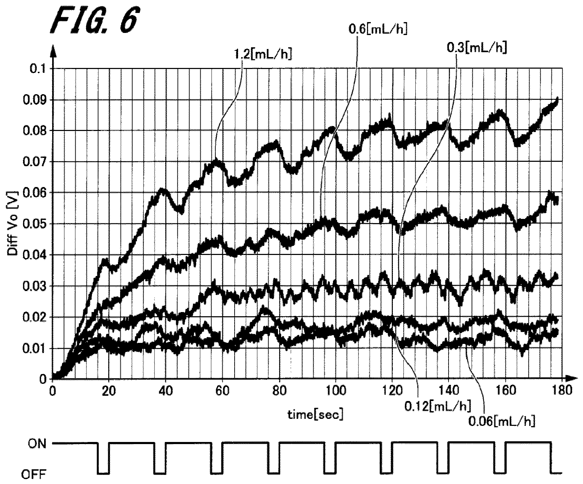

Fig. 6 shows a time variation in the output voltage V5 of the arithmetic processing unit 30 when the heat-generating element 23 is controlled in this way. In Fig. 6, the vertical axis represents the value of the voltage V5 and the horizontal axis represents time (sec.). Further, the waveform showing the ON-OFF time variation on the lower side of Fig. 6 shows the timing of turning the heat-generating element 23 on and off. In this example, a process of 20 seconds per one cycle including 16 seconds of heat generation (ON) and 4 seconds of heat dissipation (OFF) is repeated.

Fig. 6 shows examples including five steps set to 0.06 [mL/h], 0.12 [mL/h], 0.3 [mL/h], 0.6 [mL/h], and 1.2 [mL/h], as the flow rates of the pump part 13. From Fig. 6, it can be seen that the output voltage V5 increases as the flow rate increases.

The characteristic shown in Fig. 6 changes depending on the temperature of the liquid (drug solution). Also, since measurement time cannot be taken too long practically, the flow rate is determined based on the average value in the second cycle zone (20 sec. to 40 sec.) or the average value in the third cycle zone (40 sec. to 60 sec.) in the present embodiment.

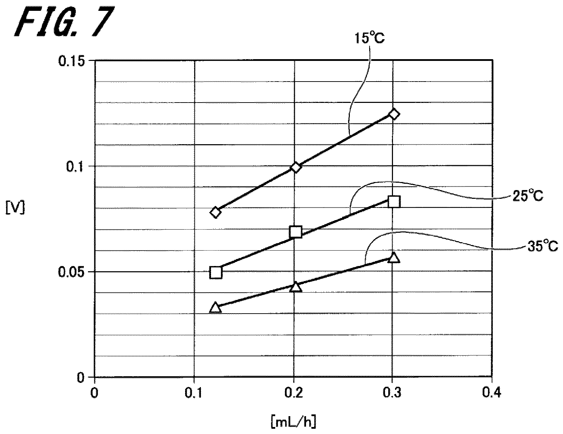

Fig. 7 is a graph that shows plotting of the average voltage value (vertical axis) in the second cycle period (or third cycle period) with respect to the flow rate (horizontal axis). In Fig. 7, examples for three temperatures of 15°C, 25°C, and 35°C are shown. As shown in Fig. 7, it can be seen that the detected voltage varies in a substantially linear manner for each liquid temperature.

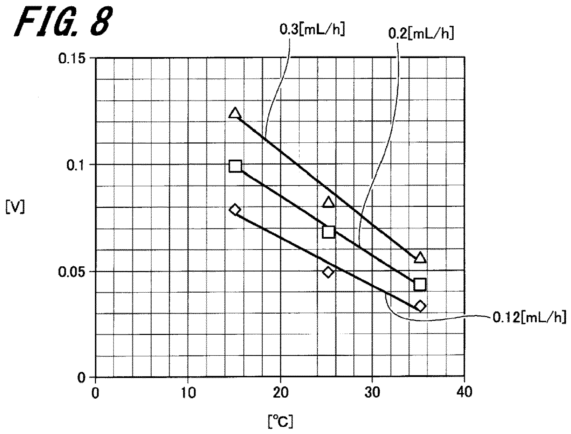

Fig. 8 is a graph showing the relationship between the liquid temperature and the average value in the third cycle zone (40 sec. to 60 sec.) for each flow rate, by using the same data as in Fig. 7. In Fig. 8, the vertical axis represents the average voltage value and the horizontal axis represents the temperature . Here, examples for three flow rates of 0.12 [mL/h], 0.2 [mL/h], and 0.3 [mL/h] are shown.

As shown in Fig. 8, it is understood that the average value of the output voltage V5 varies in a substantially linear manner with respect to the temperature for each flow rate.

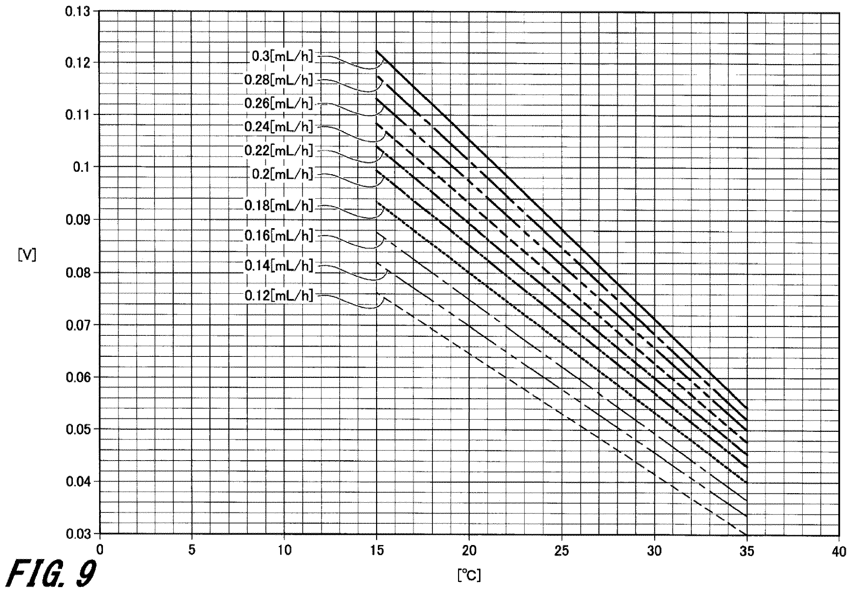

Fig. 9 shows the relationship between the average voltage value (vertical axis) and the temperature of a liquid (drug solution) (horizontal axis) at every flow rate of finely divided 10 levels (maximum 0.3 [mL/h], minimum 0.12 [mL/h]). From the relationship shown in Fig. 9, it is understood that the flow rate can be obtained from the liquid temperature and the average value of the output voltage V5.

By detecting the flow rate in the flow rate sensing part 20 in this way, a flow rate of about 100 [μL/h] at the minimum can be accurately detected.

[6. Control Example of Heat-generating Element]

It is desirable for the heat-generating element 23 to generate heat with a constant power regardless of the liquid temperature, but such heating cannot be simply performed in the case of using a thermistor. This is because the thermistor has a characteristic in which the resistance value Rt1 thereof varies depending on the liquid temperature.

In the present embodiment, the following method is used so that heat generation with constant power can be performed regardless of the liquid temperature.

Assuming that the power to be given to the thermistor is Pt, the current It satisfies

$$It = \sqrt{(Pt/Rt1)} \quad \cdots (7)$$

When this current It is given, the voltage Vin to be input is as follows based on the resistance Rin.

$$Vin = It \cdot Rin$$
$$= \sqrt{(Pt/Rt1)} \cdot Rin \quad \cdots (8)$$

That is, the input voltage Vin may be given by using the equation (8) with respect to the resistance value Rt1 which changes depending on the liquid temperature. In the equation (8), Pt and Rin are constants.

As a method for determining the resistance value Rt1 of the heat-generating element 23, any of the following processings (a) and (b) can be applied, for example.

(a) Processing example of using the resistance value measured during the heat dissipation period of the heat-generating element 23 as the resistance value Rt1 as it is

(b) Processing example of obtaining the resistance value of the heat-generating element 23 from the resistance value of the upstream-side temperature sensing element 22 or the downstream-side temperature sensing element 24 or the temperature information detected from the resistance value

To be specific, the equation (8) is a function of Rt1, and the resistance value Rt1 of the heat-generating element 23 is obtained as a voltage by the above-described processing (a) or (b), so that voltage is referred to as V6. Then, the input voltage Vin with respect to the voltage V6 is held in advance as a table, the input voltage Vin is determined from the obtained voltage V6, and the input voltage Vin is supplied to the terminal 36 (Fig. 4). In the case of the processing (b), when the heat-generating element 23, the upstream-side temperature sensing element 22 and the downstream-side temperature sensing element 24 are all thermistors, the resistance value Rt1 can be obtained directly from the outputs of the upstream-side temperature sensing element 22 and the downstream-side temperature sensing element 24, which is convenient.

[7. Example of Administration of Drug Solution]

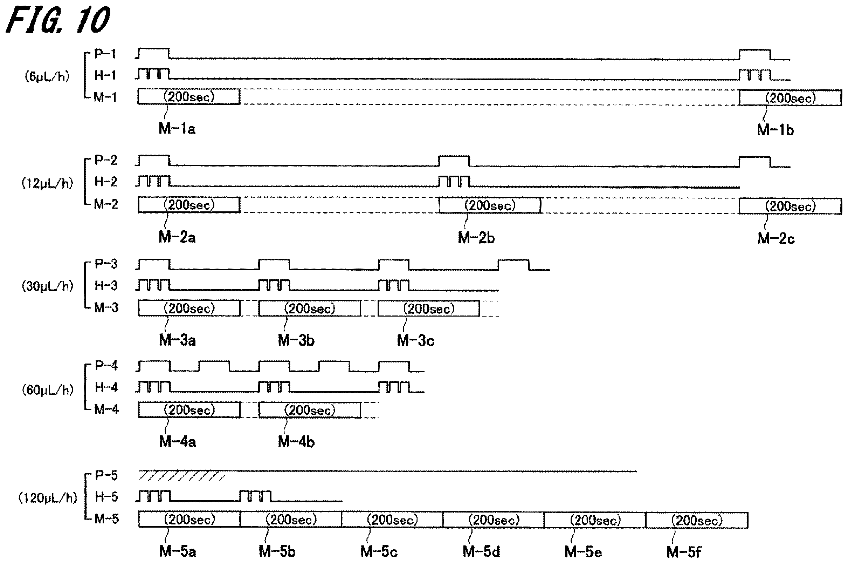

Next, processing in which the drive unit 40 controls the pump part 13 to administer the drug solution (insulin) to a patient at a low flow rate in conjunction with the flow rate detected by the flow rate sensing part 20 described above will be described with reference to Fig. 10 and Table 1.

As described above, the flow rate sensing part 20 of the present embodiment can detect a flow rate of about 100 [μL/h] at the minimum, but the minimum flow rate required for the drug-solution administration device 10 for administering insulin to a patient is about 0.5 [μL/h], which is even smaller than the minimum flow rate detected amount. When such a very low flow rate is required, the drive unit 40 achieves the required low flow rate by driving the pump part 13 intermittently.

Here, modes when insulin is administered to a patient will be described. As the modes for administering insulin to a patient, there are a basal insulin administration mode and a bolus insulin administration mode.

The basal insulin administration mode is a mode of continuously administering insulin to a patient at a very low flow rate and the insulin is administered at a very low flow rate of about 0.5 [μL/h] at the minimum.

The bolus insulin administration mode is a mode of temporarily administering insulin to a patient at a high flow rate for the purpose of controlling blood glucose level and the like after each meal, and in accordance with the amount of meal, particularly the amount of carbohydrates contained in food, insulin is administered at a flow rate of several dozen [μL/h] to about 120 [μL/h] in a short period of time.

In order to cope with each of these modes, the drug-solution administration device 10 for administering insulin is required to be capable of variably setting the amount of the drug solution administered within the range of 0.5 [μL/h] to 120 [μL/h].

Further, when starting to use the drug-solution administration device 10, a priming operation of filling the conduit line 17 with insulin is required, and a flow rate of about 300 [μL/h] is necessary in order to perform this operation in a short time.

To be specific, as the drug-solution administration device 10, it is requested that the flow rate can be set in steps of 0.5 [μL/h] in a range from a minimum flow rate of 0.5 [μL/h] to a maximum flow rate of 300 [μL/h]. Accordingly, when a flow rate equal to or lower than the flow rate that can be detected by the flow rate sensing part 20 is required, the drug-solution administration device 10 of the present embodiment intermittently administers the drug solution by the pump part 13, whereby a necessary amount of drug solution is administered.

Here, as an actual operation example of the pump part 13, the following three patterns are prepared.

•Pattern 1: (when the required flow rate is 0.5 [μL/h])

The intermittent driving in which the pump part 13 feeds the liquid at 90 [μL/h] for 40 seconds and then pauses for 119 minutes and 20 seconds is repeated.

•Pattern 2: (when the required flow rate is in the range of 1 [μL/h] to 119.5 [μL/h])

The pump part 13 repeats intermittent driving in which the pump part 13 is paused for time variably set within a range of 119 minutes to 12 seconds in accordance with the flow rate after pumping at 120 [μL/h] for 60 seconds (see Table 1).

•Pattern 3: (when the required flow rate is in the range of 120 [μL/h] to 300 [μL/h])

The drive voltage and the drive frequency of the pump part 13 are changed to perform continuous driving.

The following Table 1 shows a setting example of downtime (minutes) at the required flow rate 1 [μL/h] to 119.5 [μL/h] in the case of pattern 2. In this example of Table 1, the liquid feeding is performed at 120 [μL/h] for 60 seconds (1 minute) at any required flow rate, and the required flow rate is set by adjusting the downtime after the liquid delivery.

[Table 1]

•Set flow rate (μL/h) / liquid delivery time (minutes) / downtime (minutes)

```
1 / 1 / 119
2 / 1 / 59
3 / 1 / 39
4 / 1 / 29
5 / 1 / 23
6 / 1 / 19
8 / 1 / 14
9 / 1 / 12.333
10 / 1 / 11
12 / 1 / 9
15 / 1 / 7
16 / 1 / 6.5
18 / 1 / 5.666
20 / 1 / 5
24 / 1 / 4
25 / 1 / 3.8
30 / 1 / 3
40 / 1 / 2
50 / 1 / 1.4
60 / 1 / 1
80 / 1 / 0.5
90 / 1 / 0.333
100 / 1 / 0.2
120 / 1 / 0
```

The [Table 1] shows that [1/1/119] in the uppermost column, for example, is achieved by repeating one minute delivery at 120 [μL/h] and 119 minutes pause when the set flow rate is 1 [μL/h]. The last column [120/1/0] shows one minute delivery at 120 [μL/h] and 0 minutes pause when the set flow rate is 120 [μL/h], in other words, a set flow rate of 120 [μL/h] is achieved by continuous delivery without pause.

In the case of driving by intermittent operation, the flow rate sensing part 20 measures the flow rate during a period of liquid feeding of 40 seconds or 1 minute, and when the flow rate measured value that is obtained is higher than the target value, the drive unit 40 set the downtime to be longer than the set time (for example, the time shown in Table 1). Conversely, when the obtained flow rate measured value is lower than the target value, the drive unit 40 reduces the downtime to less than the set time (for example, the time shown in Table 1). By performing such control, very high-accuracy flow rate control having ±5% flow rate accuracy becomes possible.

Fig. 10 is a diagram showing setting examples of the driving periods P-1, P-2, P-3, P-4, and P-5 of the pump part 13, the heat generation periods H-1, H-2, H-3, H-4, and H-5 of the heat-generating element 23 (heater) in the flow rate sensing part 20, and measurement periods M-1, M-2, M-3, M-4, and M-5 of the flow rate sensing part 20.

The five examples shown in Fig. 10 are examples at flow rates of 6 [μL/h], 12 [μL/h], 30 [μL/h], 60 [μL/h], and 120 [μL/h], respectively.

The respective measurement periods of 200 seconds shown in Fig. 10 are detecting periods of one cycle in which the flow rate sensing part 20 detects the flow rate.

In principle, it is necessary for the flow rate sensing part 20 of the present embodiment to wait until the influence of heat generation disappears. For this reason, a 200 second process is performed in which a 20 second process including heat generation for 16 seconds and heat releasing for 4 seconds (suspension of heat generation) thereafter is repeated three times and then heat is released for further 140 seconds for example . The flow rate sensing part 20 periodically detects the flow rate with the period of 200 seconds as one cycle. Flow rate detection for one cycle of 200 seconds is performed intermittently or continuously for each flow rate as shown in Fig. 10.

Here, as shown in Fig. 10, intermittent driving of the pump part 13 is required depending on the target flow rate. For example, when the flow rate is 30 [μL/h] or less, the pump part 13 is driven for the first 60 seconds of the 200 seconds for which the flow rate is detected.

For example, when the flow rate is 6 [μL/h], the driving period P-1 of the pump part 13 and the heat generation period H-1 of the heat-generating element 23 are set for intermittent operation in conjunction with each other, and measurement periods M-1a, M-1b, ⋯ of 200 seconds are set in conjunction with each start of the driving period P-1 and the heat generation period H-1.

When the flow rate is 12 [μL/h], the driving period P-2 of the pump part 13 and the heat generation period H-2 of the heat-generating element 23 are set for intermittent operation at shorter intervals than at the flow rate of 6 [μL/h]. Then, measurement periods M-2a, M-2b, M-2c, ⋯ of 200 seconds are set in conjunction with each start of the driving period P-2 and the heat generation period H-2.

When the flow rate is 30 [μL/h], the driving period P-3 of the pump part 13 and the heat generation period H-3 of the heat-generating element 23 are set for intermittent operation at further shorter intervals. Then, measurement periods M-3a, M-3b, M-3c, ⋯ of 200 seconds are set in conjunction with each start of the driving period P-3 and the heat generation period H-3.

When the flow rate is 60 [μL/h], a cycle of 120 seconds is set for repeating driving and stopping of the pump part 13 every 60 seconds.

That is, when the flow rate is 60 [μL/h], a cycle of 120 seconds is set, in which the driving period P-4 of the pump part 13 is repeated every 60 seconds. The heat generation period H-4 is set for operation once every two driving periods P-4 of the pump part 13. Then, measurement periods M-4a, M-4b, ⋯ of 200 seconds are set in conjunction with each start of the heat generation periods H-4.

Further, when the flow rate is 120 [μL/h], the pump part 13 is continuously driven. In other words, when the flow rate is 120 [μL/h], the driving period P-5 of the pump part 13 is set continuous. The heat generation period H-5 of the heat-generating element 23 is set intermittent at intervals of 200 seconds. Then, measurement periods of 200 seconds M-5a, M-5b, M-5c, M-5d, M-5e, M-5f, ······ are set continuous.

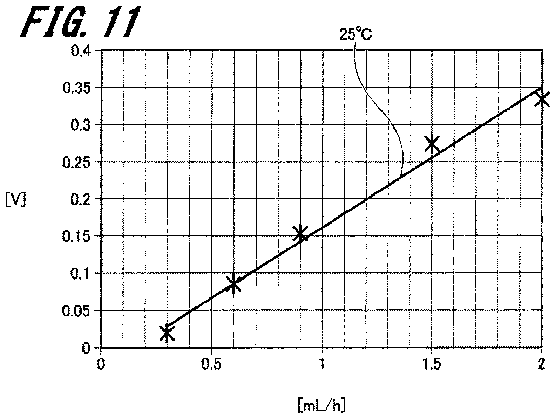

Fig. 11 shows the relationship between the voltage V5 (vertical axis) detected by the flow rate sensing part 20 and the flowrate (horizontal axis) in the case of a liquid temperature of 25°C.

As shown in Fig. 11, it becomes possible to detect the flow rate in detail based on the voltage V5 and to perform control to bring the flow rate closer to the target flow rate.

[8. Another Configuration Example of the Flow Rate Sensing Part]

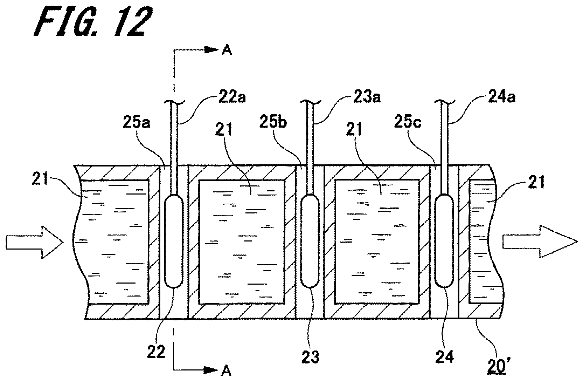

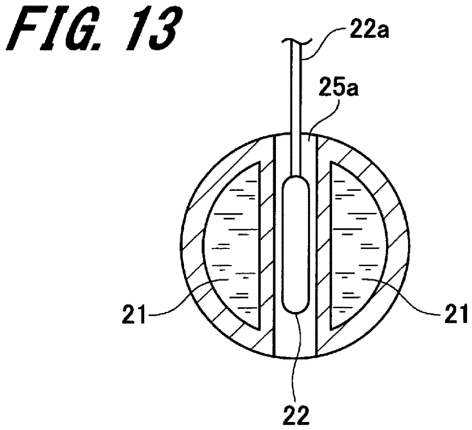

Figs. 12 and 13 show a flow rate sensing part 20' having a structure different from that of the flow rate sensing part 20 shown in Fig. 2.

The flow rate sensing part 20' shown in Fig. 12 is provided with tubular portions 25a, 25b, and 25c at equal intervals in the conduit line 21. The drug solution does not enter the tubular portions 25a, 25b, and, 25c and a drug solution is allowed to pass through the peripheries of the tubular portions 25a, 25b, and 25c as shown in Fig. 13. Fig. 13 shows a cross section of the portion where the tubular portion 25a is disposed, but the cross sections of the portions where the other tubular portions 25b and 25c are disposed have the same configuration. Each of the tubular portions 25a, 25b, and 25c is made of a material having high thermal conductivity.

Then, the first temperature sensing element (upstream-side temperature sensing element) 22 is disposed in the upstream-side tubular portion 25a, the heat-generating element 23 is disposed in the central tubular portion 25b, and a second temperature sensing element (downstream-side temperature sensing element) 24 is disposed in the downstream-side tubular portion 25c.

Although not shown in Figs. 12 and 13, a material such as a cream for improving thermal bonding is disposed between the tubular portions 25a, 25b, and 25c and the elements 23, 24, and 25, respectively.

The circuits connected to the elements 23, 24, and 25 are the same as those in the example of Figs. 3 and 4.

According to the flow rate sensing part 20' configured as described above, since the elements 23, 24, and 25 are isolated from the passage of the drug solution as compared with the flow rate sensing part 20 shown in Fig. 2, eluate entering the drug solution passing through the conduit line 21 can be reduced.

[9. Example of Improving Flow Rate Sensing Accuracy]

Next, an example of further improving the flow rate sensing accuracy when the arithmetic processing unit 30 detects the flow rate from the output voltage of the flow rate sensing part 20 will be described.

For example, the flow rate is very low at the time of basal insulin administration in which insulin is administered to a patient at a low flow rate, so that the output voltage detected by the arithmetic processing unit 30 is easily affected by an offset and drift. Processing for improving the flow rate sensing accuracy even in a situation where such an offset or drift is affecting will be described.

As already described with reference to Fig. 11 and the like, the output of the arithmetic processing unit 30 changes linearly with respect to each flow rate and shows a high correlation coefficient. In addition, the gradient of the approximate straight line shown in each figure changes linearly with respect to temperature and exhibits a high correlation coefficient.

Accordingly, a flow rate with high sensing accuracy can be obtained from the output voltage of the arithmetic processing unit 30 by the following procedures (a), (b) and (c).

(a) When the flow rate is referred to as x and the output of the arithmetic processing unit 30 is referred to as y, the following characteristic curve is obtained in advance for each temperature.

$$y = ax + b \cdots (9)$$

a is a gradient for each temperature.
Further, regarding the obtained gradient a for each temperature, the following relational expression with the temperature T is obtained.

$$a = \alpha T + \beta \cdots (10)$$

Here, α and β are constants.
(b) When the flow rate is zero before (or after) the flow rate measurement, the output y of the arithmetic processing unit 30 is measured to find the y-intercept b(T) at the temperature at the measurement time. Then, from the data of the gradient a(T) acquired in advance, the following characteristic curve at the temperature at the measurement time is determined.

$$y = a(T)x + b(T) \cdots (11)$$

(c) Calculate the flow rate x from the measured circuit output y and equation (11).

By obtaining the flow rate x in this way, the flow rate that excludes the influence of the offset and drift can be detected with high precision.

[10. Modification Example]

It is to be noted that the configuration described in the above embodiment is a preferable example, and the present invention is not limited to the configuration described in the embodiment. For example, a piezoelectric pump is used as the pump part, but a pump of other configuration may be used.

As shown in Fig. 2, with respect to each of the elements 22, 23, and 24 arranged in the conduit line 21 constituting the flow rate sensing part 20, some coating may be applied to the surface to reduce the amount of eluate entering a drug solution passing through the conduit line 21.

Further, in the above-described embodiment, the measurement of the liquid temperature in the conduit line is performed by the heat-generating element itself by utilizing the non-heat generating period of the heat-generating element. In contrast, a dedicated temperature sensing element for measuring the liquid temperature inside the conduit line may be provided.

For example, liquid temperature may be measured by disposing an element (such as a thermistor) for measuring the liquid temperature further upstream of the first temperature sensing element 22 shown in Fig. 2 or 12 to reduce the influence of heat generation by the heat-generating element. By doing in this manner, it becomes possible to conduct administration at lower flow rate with higher accuracy.

Further, in the above-described embodiment, although the case of application to the portable insulin administration device has been described, the present invention may be applied to a drug-solution administration device for administering drug solutions other than insulin. In addition, a configuration as a portable type in a small size is one example, and the configuration may be a stationary type.

Reference Signs List

    10 drug-solution administration device
    11 reservoir
    12, 14, 16, 17, 21 conduit line
    13 pump part
    15 microneedle valve
    20, 20' flow rate sensing part
    21a, 21b, 21c element disposing position
    22 first temperature sensing element (upstream-side temperature sensing element)
    22a terminal
    23 heat-generating element
    23a terminal

    24 second temperature sensing element (downstream-side temperature sensing element)
    24a terminal
    25a, 25b, 25c tubular portion
    30 arithmetic processing unit
    31 first voltage conversion circuit
    32 second voltage conversion circuit
    33 differential amplifier circuit
    34 low-pass filter
    31a, 32a, 33a, 34a operational amplifier
    35 output terminal
    36 terminal
    37 operational amplifier
    38 terminal
    40 drive unit
    C1 capacitor
    P1 characteristic of flow rate of a single unit
    P2 fluctuation of flow rate at output portion
    R1, R2, R3, R5, R6, R8, R9, R12, R14, R17, R19, R21, Rin resistor

**Claims**

1. A device for administering a drug solution comprising:

    a pump part which delivers and administers a drug solution held in a reservoir to a live body;
    a flow rate sensing part which detects a flow rate of the delivered solution generated by the pump part; and
    a drive unit which controls a drive state of the pump part based on the flow rate detected by the flow rate sensing part, wherein
    the flow rate sensing part includes:

       a heat-generating element disposed in a conduit line in which the drug solution is delivered;
       a first temperature sensing element disposed in the conduit line on an upstream side of the heat-generating element;
       a second temperature sensing element disposed in the conduit line on a downstream side of the heat-generating element; and
       an arithmetic processing unit which calculates the flow rate in the conduit line based on a difference between temperatures detected by the first temperature sensing element and the second temperature sensing element.

2. The device for administering a drug solution according to claim 1, wherein
   the heat-generating element repeats a heat generation state and a non-heat generation state for each fixed period of time, and

the arithmetic processing unit calculates the flow rate during the fixed period from an average value of the difference between temperatures detected by the first temperature sensing element and the second temperature sensing element during the fixed period.

3. The device for administering a drug solution according to claim 2, wherein
the heat-generating element is an element which generates heat by voltage application and whose resistance value varies with temperature, and
the device prevents a temperature rise of the drug solution equal to or higher than a threshold value based on temperature information detected from the resistance value of the heat-generating element during the non-heat generation state.

4. The device for administering a drug solution according to claim 3, wherein
the device sets electric power to constant power during a period when the heat-generating element is in the heat generation state based on temperature information detected by the first temperature sensing element and the second temperature sensing element or temperature information detected from the resistance value of the heat-generating element.

5. The device for administering a drug solution according to claim 1, further comprising a third temperature sensing element on an upstream side of the first temperature sensing element.

6. The device for administering a drug solution according to claim 1, wherein
the pump part includes a piezoelectric pump which drives the piezoelectric element with a predetermined frequency and voltage.

7. The device for administering a drug solution according to claim 1, wherein
the first temperature sensing element and the second temperature sensing element are arranged at an equal distance from the heat-generating element.

8. The device for administering a drug solution according to claim 1, wherein
the heat-generating element, the first temperature sensing element, and the second temperature sensing element are provided with a predetermined coating on each surface and disposed in the conduit line.

9. The device for administering a drug solution according to claim 1, wherein
the heat-generating element, the first temperature sensing element, and the second temperature sensing element are disposed in contact with an area through which a drug solution passes in the conduit line via a partition wall.

**11**

FIG. 1

EP 3 351 286 A1

*FIG. 2*

22a  23a  24a
21a  21b  21c
21

22  23  24

20

FIG. 3

# FIG. 4

## FIG. 5

FIG. 6

## FIG. 7

FIG. 8

FIG. 9

FIG. 10

FIG. 11

# FIG. 12

FIG. 13

## INTERNATIONAL SEARCH REPORT

International application No.

PCT/JP2016/071271

**A. CLASSIFICATION OF SUBJECT MATTER**
*A61M37/00*(2006.01)i, *A61M5/142*(2006.01)i, *A61M5/168*(2006.01)i

According to International Patent Classification (IPC) or to both national classification and IPC

**B. FIELDS SEARCHED**

Minimum documentation searched (classification system followed by classification symbols)
A61M37/00, A61M5/142, A61M5/168

Documentation searched other than minimum documentation to the extent that such documents are included in the fields searched
Jitsuyo Shinan Koho          1922-1996   Jitsuyo Shinan Toroku Koho   1996-2016
Kokai Jitsuyo Shinan Koho    1971-2016   Toroku Jitsuyo Shinan Koho   1994-2016

Electronic data base consulted during the international search (name of data base and, where practicable, search terms used)
WPI

**C. DOCUMENTS CONSIDERED TO BE RELEVANT**

| Category* | Citation of document, with indication, where appropriate, of the relevant passages | Relevant to claim No. |
|---|---|---|
| Y | JP 2011-527749 A  (Koninklijke Philips Electronics N.V.), 04 November 2011 (04.11.2011), paragraphs [0001] to [0005], [0009] to [0010], [0020] to [0021], [0034] to [0047]; fig. 1 to 5 & US 2011/0118705 A1 fig. 1 to 5; paragraphs [0001] to [0005], [0009] to [0010], [0020] to [0021], [0041] to [0059] & WO 2010/004484 A1      & CN 102089632 A | 1-9 |

☒  Further documents are listed in the continuation of Box C.          ☐  See patent family annex.

| | |
|---|---|
| *   Special categories of cited documents: | "T"  later document published after the international filing date or priority date and not in conflict with the application but cited to understand the principle or theory underlying the invention |
| "A"  document defining the general state of the art which is not considered   to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| 12 October 2016 (12.10.16) | 25 October 2016 (25.10.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

| International application No. |
|---|
| PCT/JP2016/071271 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2012-228481 A  (Terumo Corp.),<br>22 November 2012 (22.11.2012),<br>paragraphs [0011] to [0013], [0056] to [0069];<br>fig. 4 to 7<br>& EP 2700424 A1<br>paragraphs [0012], [0020], [0144] to [0172];<br>fig. 16 to 19<br>& WO 2012/144219 A1     & CN 103491996 A | 1-9 |
| A | US 5533412 A  (IC SENSORS, INC., BAXTER<br>INTERNATIONAL INC.),<br>09 July 1996 (09.07.1996),<br>fig. 1a; column 4, line 43 to column 5, line 33<br>& WO 1995/002164 A1 | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002126092 A